# EUROPÄISCHE PATENTSCHRIFT

(11) **EP 0 675 942 B1**
(45) Veröffentlichungstag und Bekanntmachung des Hinweises auf die Patenterteilung: **30.07.1997**
(21) Anmeldenummer: 93910006.1
(22) Anmeldetag: 17.05.1993
(51) Int. Cl.: C11D 1/72, D06L 1/14, D06L 3/02

(54) **NETZMITTEL FÜR DIE TEXTILE VORBEHANDLUNG**
WETTING AGENT FOR TEXTILE CONDITIONING
AGENTS MOUILLANTS POUR LE PRETRAITEMENT TEXTILE

(30) Priorität: 22.12.1992 DE 4243643
(43) Veröffentlichungstag der Anmeldung: 11.10.1995
(73) Patentinhaber: Henkel Kommanditgesellschaft auf Aktien, 40191 Düsseldorf (DE)
(72) Erfinder: SCHMID, Karl-Heinz, D-4020 Mettmann (DE); WEGENER, Ingo, D-4000 Düsseldorf 13 (DE); HANKE, Anja, D-4100 Duisburg 25 (DE); NEUSS, Michael, D-5000 Köln 20 (DE); RATHS, Hans-Christian, D-40789 Monheim (DE)
(86) Internationale Anmeldenummer: EP9301226
(87) Internationale Veröffentlichungsnummer: WO9414936

(56) Entgegenhaltungen:
- EP-A- 0 161 537
- EP-A- 0 420 802

## Beschreibung

### Gebiet der Erfindung

Die Erfindung betrifft Netzmittel für die textile Vorbehandlung, die einen Gehalt an ausgewählten Mischethern aufweisen, sowie die Verwendung der Mischether zur Herstellung von Netzmitteln für die textile Vorbehandlung.

### Stand der Technik

Bei der Vorbehandlung von Garnen und Geweben werden Netzmittel eingesetzt, zu deren Aufgabe es beispielsweise gehört, die Ablösung von Verunreinigungen von den Fasern oder die Bleiche zu unterstützen. An Hilfsmittel dieser Art werden umfangreiche Anforderungen gestellt. So müssen sie beispielsweise unter den häufig stark alkalischen Bedingungen chemisch stabil sein, eine Benetzungszeit im Sekundenbereich aufweisen, keinen eigenen Schaum liefern, Prozeßschaum unterdrücken und zudem biologisch leicht abbaubar sein.

Aus dem Stand der Technik sind bereits eine große Zahl von Dokumenten bekannt, die Fettalkoholpolyglycolether und insbesondere endgruppenverschlossene Fettalkoholpolyglycolether, sogenannte "Mischether", als Problemlösungen für diese oder ähnliche Fälle vorschlagen:

So sind beispielsweise aus der **EP-A 0 124 815** (Henkel) Mischether mit 8 bis 18 Kohlenstoffatomen in der Fettalkyl- und 7 bis 12 Ethylenoxideinheiten in der Polyetherkette als schaumdrückende Zusätze zu schaumarmen Reinigungsmitteln bekannt. Für den gleichen Zweck werden in der **EP-B 0 303 928** (Henkel) Octyl- und/oder Decylmischether mit 3 bis 4 Ethylenoxid-Einheiten vorgeschlagen.

Für die Schaumdämpfung bei der Verarbeitung von Nahrungsmitteln sowie bei Fermentationsprozessen werden in der **EP-A 0 180 081** (BASF) Mischether mit 6 bis 12 Kohlenstoffatomen im Fettalkylrest und EO/PO/EO-Blöcken in der Polyetherkette vorgeschlagen. Gemäß der Lehre der **EP-B 0 324 340** (Henkel) können für diesen Zweck auch Mischether mit 6 bis 28 Kohlenstoffatomen im Fettalkylrest und 2 bis 10 Ethylenoxideinheiten in der Polyetherkette eingesetzt werden.

Schließlich werden in der **EP-A 0 420 802** (Ciba-Geigy) Netzmittel für die textile Vorbehandlung vorgeschlagen, die einen Gehalt an offenkettigen und/oder endgruppen-verschlossenen Fettalkoholpolyglycolethern aufweisen. Als geeignete Einsatzstoffe werden solche offenbart, die einen Fettalkylrest von mindestens 8, vorzugsweise 9 bis 14 Kohlenstoffatomen und 2 bis 24, vorzugsweise 4 bis 8 Alkylenoxid-Einheiten in der Polyetherkette aufweisen; sie können offenkettig oder aber mit einer C₁-C₈ Alkylgruppe, einem cycloaliphatischen Rest mit mindestens 5 Kohlenstoffatomen sowie einem Phenylniederalkyl- oder Styrylrest endgruppenverschlossen sein. Gemäß dem einzigen Ausführungsbeispiel wird ausschließlich die Verwendung eines offenkettigen Anlagerungsproduktes von 15 Mol Alkylenoxid an einen C₉-C₁₁-Oxoalkohol offenbart.

Alle diese Schriften ofenbaren eine große Zahl von möglichen Mischethertypen und deren vorteilhafte Verwendung als Schaumregulierungs- oder Netzmittel. Kein einziges dieser Dokumente nimmt jedoch Bezug auf die vorteilhafte Kombination der verschiedenen erforderlichen Parameter oder bietet dem Fachmann eine Lehre, nach welchen Kriterien er die Strukturelemente - Fettalkylrest, Propoxylierungsgrad, Ethoxylierungsgrad, Endgruppenverschluß - kombinieren muß, um zu Mischethertypen zu gelangen, die der erwähnten kumulierten Aufgabenstellung gerecht werden.

Die Aufgabe der Erfindung bestand somit darin, neue Netzmittel zu entwickeln, die gleichzeitig schaumarm, schaummindernd, rasch netzend, alkalibeständig und biologisch leicht abbaubar sind.

### Beschreibung der Erfindung

Gegenstand der Erfindung sind Netzmittel für die textile Vorbehandlung mit einem Gehalt an Mischethern der Formel (I), 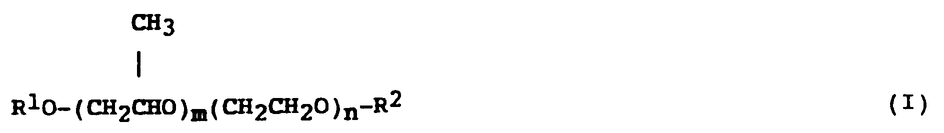 in der R¹ für einen streng linearen Alkylrest mit 8 bis 10 Kohlenstoffatomen, R² für einen Alkylrest mit 1 bis 4 Kohlenstoffatomen, m für Zahlen von 0,5 bis 2 und n für Zahlen von 6 bis 9 steht.

Überraschenderweise wurde gefunden, daß unter der Vielzahl der als schaumarme Netzmittel bekannten Mischether gerade ein eng begrenzter Bereich von Species die für die textile Vorbehandlung erforderlichen Parameter - nämlich extreme Schaumarmut, Dämpfung des Prozeßschaums, hohes Netzvermögen, Alkalibeständigkeit und ausgezeichnete biologische Abbaubarkeit - in vorzüglicher Weise erfüllen. Die Produkte sind zudem äußerst lagerstabil.

Die vorliegenden Beispiele und Vergleichsbeispiele zeigen, daß bereits eine geringe Modifizierung der Länge der Fettalkylkette oder des Ethoxylierungsgrades eine signifikante Verschlechterung des Netz- und Schaumvermögens bewirkt, während eine Steigerung des Propylenglycolgehaltes oder das Einführen von Verzweigungen in den Fettalkylrest die biologische Abbaubarkeit der Produkte sowie deren schaumdrückende Wirkung drastisch verschlechtert.

### Mischether

**Mischether** stellen bekannte Stoffe dar, die nach den einschlägigen Verfahren der präparativen organischen Chemie erhalten werden können. Üblicherweise erfolgt ihre Herstellung über den Weg der WILLIAMSON'schen Ethersynthese, bei dem man Fettalkoholpolyglycolether in Gegenwart starker Basen mit Alkylhalogeniden kondensiert. Verfahren zu ihrer Herstellung sind beispielsweise aus den Druckschriften **DE-OS 28 00 710** (Kuraray) und **DE-C1 37 44 525** (Henkel). Eine Übersicht von Piorr et al. zum Thema "Mischether" findet sich ferner in **Fat Sci.Technol. 89, 106 (1987).**

Typische Beispiele für Mischether, die als Bestandteile der erfindungsgemäßen Netzmittel in Frage kommen, sind methyl-, ethyl- oder butyl-endgruppenverschlossene Anlagerungsprodukte von 0,5 bis 2 Mol Propylenoxid und 6 bis 9 Mol Ethylenoxid an Octanol und/oder Decanol. Aus anwendungstechnischer Sicht bevorzugt sind Mischether der Formel (I), in der R¹ für einen Octylrest, R⁴ für einen n-Butylrest, m für Zahlen von 1,2 bis 1,7 und n für Zahlen von 6 bis 8 und insbesondere 7 bis 8 stehen, ideal. Im Hinblick auf Schaumarmut, Netzvermögen und biologische Abbaubarkeit ist ein Mischether hervorzuheben, der durch Anlagerung von zunächst 1,2 Mol Propylenoxid und dann 6 bzw. 7 Mol Ethylenoxid an Octanol und nachfolgenden Endgruppenverschluß mit Butylchlorid zugänglich ist.

Der Begriff "streng linear" ist in diesem Zusammenhang so zu verstehen, daß der Gehalt an verzweigten Species im Fettalkylrest 0,5 Gew.-% nicht übersteigen darf. Damit ist klar gestellt, daß Mischether auf Basis von Oxoalkoholen, deren Anteil an verzweigten Homologen üblicherweise im Bereich von 5 bis 25 Gew.-% liegt, für die erfindungsgemäßen Netzmittel nicht in Betracht kommen.

Die erfindungsgemäßen Netzmittel können die Mischether der Formel (I) in Mengen von 5 bis 90, vorzugsweise 50 bis 85 Gew.-% - bezogen auf die Mittel - enthalten. Des weiteren können die erfindungsgemäßen Mittel weitere übliche Zusatzstoffe, insbesondere Hydrotrope und Lösungsmittel enthalten.

### Hydrotrope

a1) Als Hydrotrope kommen lineare oder verzweigte, **aliphatische Alkohole** mit 4 bis 8 Kohlenstoffatomen in Betracht. Typische Beispiele sind Ethanol, Propanol, Butanol, Pentanol, Capronalkohol und 2-Ethylhexanol.
a2) Als weitere Hydrotrope können auch **cycloaliphatische Alkohole** vom Typ der Terpenole berücksichtigt werden, wie beispielsweise Geraniol, Dihydroterpinol, Nopol, Myrcenol und Terpineol.
a3) Des weiteren kommen als Hydrotrope **aromatische Alkohole** wie beispielsweise Benzylalkohol, Phenylethanol, Phenoxyethanol, 1-Phenoxy-2-propanol (Phenoxyisopropylalkohol) und Zimtalkohol in Frage.
a4) Aus der Gruppe der **anionischen Tenside** können Sulfonate des Camphers, Toluols, Xylols, Cumols und Naphthols eingesetzt werden. Des weiteren geeignet sind Sulfate von Fettalkoholen mit 6 bis 22 Kohlenstoffatomen, wie beispielsweise Capronalkohol, Caprylalkohol, 2-Ethylhexylalkohol, Caprinalkohol, Laurylalkohol, Myristylalkohol, Cetylalkohol, Palm- oleylalkohol, Stearylalkohol, Isostearylalkohol, Oleylalkohol, Elaidylalkohol, Petroselinylalkohol, Linolylal- kohol, Linolenylalkohol, Elaestearylalkohol, Arachylalkohol, Gadoleylalkohol, Behenylalkohol und Erucylalkohol sowie deren technische Gemische, wie sie beispielsweise bei der Hydrierung von technischen Methylesterfraktionen oder Aldehyden aus der Roelen'schen Oxosynthese anfallen. Die Tenside können in Form ihrer Alkali-, insbesondere ihrer Natrium- bzw. Kaliumsalze eingesetzt werden.
a5) Weiterhin als Hydrotrope geeignet sind **Di- bzw. Polycarbonsäuren,** wie beispielsweise Malonsäure, Bernsteinsäure, Glutarsäure, Adipinsäure, Pimelinsäure, Korksäure, Azelainsäure, Sebacinsäure, Fumarsäure, Maleinsäure, Weinsäure, Äpfelsäure, Citronensäure und Aconitsäure sowie Polyacrylsäuren und Alkylphosphonsäuren. Die Säuren können in freier Form, aber auch in Form ihrer Alkalisalze bzw. Partialester eingesetzt werden.
a6) Als Hydrotrope kommen auch **nichtionische Tenside,** insbesondere Anlagerungsprodukte von durchschnittlich 5 bis 10 Mol Ethylenoxid and Fettalkohole mit 6 bis 18 Kohlenstoffatomen in Betracht, die eine konventionelle oder eingeengte Homologenverteilung aufweisen können. Typische Beispiele sind Addukte von 4 Mol Ethylenoxid an Octanol oder 5 Mol Ethylenoxid an Decanol. Weitere geeignete nichtionische Tenside stellen insbesondere Alkyloligoglucoside und Fettsäure-N-alkylglucamide dar. Typische Beispiele für letztgenannte Stoffklassen sind C_{12/14}-Kokosalkylglucosid und Laurinsäure-N-methylglucamid.

Der Anteil der Hydrotrope an den erfindungsgemäßen Mitteln kann üblicherweise 1 bis 25, vorzugsweise 5 bis 20 Gew.-% betragen.

### Lösungsmittel

Als Lösungsmittel kommen vorzugsweise solche unpolaren Stoffe in Betracht, die einen Flammpunkt oberhalb von 65°C aufweisen. Typische Beispiele sind Cyclohexanol, Methylcyclohexanol, Tetralin und 3,5,5-Trimethylhexanol. Ferner können als unpolare organische Lösungsmittel Ester wie beispielsweise Tributylcitrat oder Tributylphosphat eingesetzt werden. Der Anteil der Lösungsmittel an den erfindungsgemäßen Mitteln kann üblicherweise 0 bis 10, vorzugsweise 2 bis 8 Gew.-% betragen.

### Netzmittel

Die erfindungsgemäßen Netzmittel können durch Eintragen der Mischether sowie gegebenenfalls der übrigen Inhaltsstoffe in Wasser hergestellt werden. Es handelt sich um einen rein mechanischen Vorgang, der durch Rühren und erhöhte Temperatur (30 bis 40°C) unterstützt wird; eine chemische Reaktion findet nicht statt.

Eine typische Netzmittelformulierung weist beispielsweise folgende Zusammensetzung auf:

| | |
|---|---|
| Mischether (Octanol-1,2PO/6EO-butylether) | 30 Gew.-% |
| 2-Ethylhexylsulfat-Na-Salz (30 gew.-%ige Lösung) | 10 Gew.-% |
| Decylalkohol-5 EO | 5 Gew.-% |
| 3,5,5-Trimethylhexanol | 5 Gew.-% |
| Wasser | 50 Gew.-% |

Die Einsatzmenge, in denen die erfindungsgemäßen Netzmittel den Behandlungsflotten zugesetzt werden, kann zwischen 1 und 10, vorzugsweise 0,5 und 5 g pro Liter Flotte betragen. Naturgemäß kann die Flotte noch weitere Zusätze enthalten, z. B. Farbstoffe, optische Aufheller, Alkalien, wie etwa Natriumhydroxid, Sequestierungsmittel wie Phosphonate und Wasserglas.

### Gewerbliche Anwendbarkeit

Die erfindungsgemäßen Netzmittel sind schaumfrei, rasch netzend, hoch-alkalibeständig, lagerstabil und leicht biologisch abbaubar. Sie dämpfen den Prozeßschaum und eignen sich daher zur Herstellung von Mitteln für die textile Vorbehandlung. Hierunter sind insbesondere die alkalische Vorbehandlung sowie die Chlor- und Peroxid-Bleiche von Rohbaumwolle zu verstehen. Gleichfalls in Betracht kommt auch die übliche Vorbehandlung - wie etwa das Reinigen oder Färben aus einem Bad - von Fasern und Fasergemischen wie beispielsweise Cellulose, Wolle, Polyamid-, Polyacrylnitril- oder Polyesterfasermaterialien sowie Polyacryl/Baumwoll- und Polyester/Baumwoll-Geweben.

Der Kern der vorliegenden Erfindung besteht in der erfinderischen und durch den Stand der Technik nicht nahegelegten Auswahl kurzkettiger, im Fettalkylrest streng linearer Mischether mit niedrigem Gehalt an Propylen- und Ethylenoxideinheiten aus der großen Zahl der für diese oder ähnliche Anwendungen bereits bekannten Mischether.

Ein weiterer Gegenstand der Erfindung betrifft daher die Verwendung von Mischethern der Formel (I) zur Herstellung von Netzmitteln für die textile Vorbehandlung, in denen sie in Mengen von 5 bis 90, vorzugsweise 50 bis 85 Gew.-% - bezogen auf die Mittel - enthalten sein können.

Die folgenden Beispiele sollen den Gegenstand der Erfindung näher erläutern, ohne ihn darauf einzuschränken.

### Beispiele

### Anwendungstechnische Prüfungen

### Beispiel 1 bis 10, Verageichsbeispiele V1 bis V9:

### 1) Netzvermögen

Das Netzvermögen wurde nach der Tauchnetzmethode mit 1 g Aktivsubstanz/l, 20°C, in Wasser von 16°d bestimmt. Zu Einzelheiten der Methode sei auf **Tens.Surf.Det. 27, 243 (1990)** verwiesen. Bestimmt wurde die Netzzeit tₙ in s.

### 2) Schaumvermögen

Das Schaumvermögen wurde nach der Schlagschaummethode mit 1 g Aktivsubstanz/l, 20°C, in Wasser von 16°d bestimmt. Zu Einzelheiten der Methode sei auf **Tens.Surf. Det. 27, 243 (1990)** verwiesen. Bestimmt wurde der Basisschaum (t=0) und der Schaumzerfall nach 5 min in ml.

### 3) Biologische Abbaubarkeit

Die Biologische Abbaubarkeit wurde nach der Methode des Geschlossenen Flaschentests untersucht. Bestimmt wurde die Abbaurate BSB/COD nach 30 Tagen gegen den Standard C_{12/18}-Fettalkohol-10-EO-butylether (100 %). Zu Einzelheiten vgl. **Fette, Seifen, Anstrichmitt., 87, 421 (1985).**

Die Ergebnisse sind in Tab.1 zusammengefaßt.

**Tab.1:**

| Anwendungstechnische Prüfung von Mischethern | | | | | | | | |
|---|---|---|---|---|---|---|---|---|
| Bsp. | R¹ | R² | m | n | tₙ s | Schaum (ml) | | BSB/CSB %rel |
| | | | | | | 0' | 5' | |
| 1 | C₈ | C₄ | 1,2 | 7 | 20 | 0 | 0 | 107 |
| 2 | C₈ | C₄ | 1,2 | 8 | 18 | 0 | 0 | 107 |
| 3 | C₈ | C₄ | 1,2 | 9 | 19 | 0 | 0 | 106 |
| 4 | C₈ | C₄ | 1,5 | 8 | 18 | 0 | 0 | 105 |
| 5 | C₈ | C₄ | 1,7 | 8 | 18 | 0 | 0 | 101 |
| 6 | C_{8/10}* | C₄ | 1,2 | 7 | 22 | 0 | 0 | 107 |
| 7 | C_{8/10} | C₄ | 1,2 | 8 | 21 | 0 | 0 | 107 |
| 8 | C_{8/10} | C₄ | 1,2 | 9 | 21 | 0 | 0 | 107 |
| 9 | C_{8/10} | C₄ | 1,5 | 8 | 21 | 0 | 0 | 105 |
| 10 | C_{8/10} | C₄ | 1,7 | 9 | 21 | 0 | 0 | 100 |
| V1 | C₈ | C₄ | 1,2 | 4 | 23 | 50 | 40 | 103 |
| V2 | C₈ | C₄ | 1,2 | 5 | 23 | 30 | 20 | 102 |
| V3 | C₈ | C₄ | 1,2 | 6 | 26 | 40 | 30 | 99 |
| V4 | C₈ | C₄ | 1,2 | 10 | 28 | 50 | 40 | 97 |
| V5 | C_{9/11}^{#} | C₄ | 1,2 | 8 | 20 | 30 | 20 | 89 |
| V6 | C_{12/14}⁺ | C₄ | 1,2 | 7 | 23 | 10 | 5 | 100 |
| V7 | C_{12/14} | C₄ | 1,5 | 7 | 23 | 5 | 0 | 99 |
| V8 | C_{12/14} | C₄ | 1,2 | 8 | 24 | 10 | 5 | 100 |
| V9 | C_{12/14} | C₄ | 1,5 | 9 | 24 | 5 | 0 | 98 |

| | | | | | | | | |
|---|---|---|---|---|---|---|---|---|
| Legende: *) Basis C_{8/10}-Vorlauf-Fettalkohol | | | | | | | | |
| #) Basis C_{9/11}-Oxoalkohol (35 Gew.-% verzw.Anteile) | | | | | | | | |
| +) Basis C_{12/14}-Kokosfettalkohol | | | | | | | | |

### Beispiel 11, Vergleichsbeispiele V11 und V12:

Die schaumdämpfende Wirkung der Netzmittel wurde nach der Freifallkreislaufmethode bestimmt. Untersucht wurde die Schaumentwicklung von 1 g/l Resolinrot allein sowie unter Zusatz von jeweils 1 g/l Mischether. Die Testmischungen wurden dabei innerhalb von t = 30 min von T = 20 bis auf 54°C aufgeheizt. Die Ergebnisse sind in Tab.2 zusammengefaßt.

**Tab.2:**

| Schaumdämpfung von Mischethern | | | | | | | |
|---|---|---|---|---|---|---|---|
| Bsp. | R¹ | R² | m | n | t min | T °C | Schaumhöhe ml |
| 11 | C₈ | C₄ | 1,2 | 6 | 0 | 20 | 100 |
| | | | | | 1 | 21 | 100 |
| | | | | | 5 | 26 | 90 |
| | | | | | 10 | 31 | 80 |
| | | | | | 20 | 44 | 80 |
| | | | | | 30 | 54 | 80 |
| V11 | ohne Mischether | | | | 0 | 20 | 300 |
| | | | | | 30 | 54 | 300 |
| V12 | C_{9/11} | C₄ | 1,2 | 8 | 0 | 20 | 300 |
| | | | | | 1 | 21 | 310 |
| | | | | | 5 | 26 | 340 |
| | | | | | 10 | 31 | 350 |
| | | | | | 20 | 44 | 340 |
| | | | | | 30 | 54 | 330 |

## Patentansprüche

1. Netzmittel für die textile Vorbehandlung mit einem Gehalt an Mischethern der Formel (1), 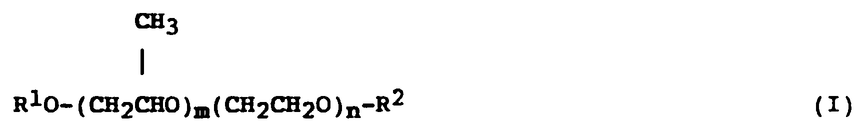 in der R¹ für einen streng linearen Alkylrest mit 8 bis 10 Kohlenstoffatomen, R² für einen Alkylrest mit 1 bis 4 Kohlenstoffatomen, m für Zahlen von 0,5 bis 2 und n für Zahlen von 6 bis 9 steht.

2. Netzmittel nach Anspruch 1, **dadurch gekennzeichnet,** daß R¹ für einen n-Octylrest, R⁴ für einen n-Butylrest, m für Zahlen von 1,2 bis 1,7 und n für Zahlen von 6 bis 8 steht.

3. Netzmittel nach den Ansprüchen 1 und 2, **dadurch gekennzeichnet,** daß Mischether der Formel **(I)** in Mengen von 5 bis 90 Gew.-% - bezogen auf die Mittel - enthält.

4. Verwendung von Mischethern der Formel **(I)** zur Herstellung von Netzmitteln für die textile Vorbehandlung.

## Claims

1. Wetting agents for the pretreatment of textiles containing mixed ethers corresponding to formula **(I):** 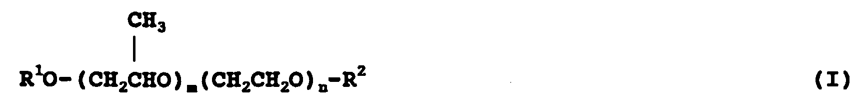 in which R¹ is a strictly linear alkyl radical containing 8 to 10 carbon atoms, R² is an alkyl radical containing 1 to 4 carbon atoms, m is a number of 0.5 to 2 and n is a number of 6 to 9.

2. Wetting agents as claimed in claim 1, **characterized in that** R¹ is an n-octyl radical, R⁴ is an n-butyl radical, m is a number of 1.2 to 1.7 and n is a number of 6 to 8.

3. Wetting agents as claimed in claims 1 and 2, **characterized in that** mixed ethers corresponding to formula **(I)** are present in quantities of 5 to 90% by weight, based on the wetting agent.

4. The use of mixed ethers corresponding to formula **(I)** for the production of wetting agents for the pretreatment of textiles.

## Revendications

1. Agent mouillant pour le prétraitement du textile contenant des éthers mixtes de formule (I), 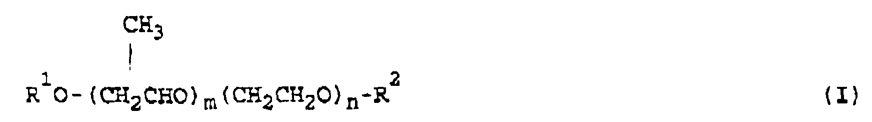 dans laquelle R¹ représente un radical alkyle strictement linéaire ayant de 8 à 10 atomes de carbone, R² représente un radical alkyle ayant de 1 à 4 atomes de carbone, m représente des chiffres allant de 0,5 à 2 et n représente des chiffres de 6 a 9.

2. Agent mouillant selon la revendication 1,
caractérisé en ce que
R¹ représente un radical n-octyle, R⁴ représente un radical n-butyle, m représente des nombres allant de 1, 2 à 1, 7 et n représente des nombres allant de 6 à 8.

3. Agent mouillant selon les revendications 1 et 2,
caractérisé en ce qu'
il contient l'éther mixte de formule (I) en quantités allant de 5 à 90 % en poids rapporté a l'agent.

4. Utilisation d'éthers mixtes de formule (I) en vue de la production d'agents mouillants pour le prétraitement du textile.
